# EUROPEAN PATENT APPLICATION

(11) **EP 1 679 032 A1**
(43) Date of publication of application: **12.07.2006**
(21) Application number: 05028685.5
(22) Date of filing: 29.12.2005
(51) Int. Cl.: A61B 5/0235, A61H 1/00, A61M 39/22, A61M 16/20, F16K 31/02

(54) **Air valve with a dielectric elastomer actuator**

(30) Priority: 07.01.2005 JP 2005002618
(71) Applicant: Omron Healthcare Co., Ltd., Kyoto 615-0084 (JP)
(72) Inventor: Kishimoto, Hiroshi, Ukyo-ku Kyoto-shi Kyoto 615-0084 (JP); Sano, Yoshihiko, Ukyo-ku Kyoto-shi Kyoto 615-0084 (JP); Karo, Hiromichi, Ukyo-ku Kyoto-shi Kyoto 615-0084 (JP)
(74) Representative: Kilian, Helmut

(57) **Abstract**

An air valve comprising: an inflow opening through which air flows in; an outflow opening through which the entered air flows out; and an actuator having an elastomer or polymer that can expand and contract when supplied with a voltage and an electrode for applying a voltage on the elastomer or polymer, wherein an outflow route from the inflow opening to the outflow opening is opened and closed by driving the actuator.

## Description

### BACKGROUND OF THE INVENTION

### Field of the Invention

The present invention relates to an air valve. More specifically, it relates to an electronic blood pressure monitor and an air massager that use the air valve.

### Description of the Related Art

Conventionally, a blood pressure of a man has been measured by winding an airbag (cuff) around his body (upper arm or wrist) and sending air into the airbag by using an air pump to obtain information of an arterial pulse wave that is sensed due to compression of the human body. Then, by depressurizing the airbag gradually to decompress the human body, measurement is finished. Therefore, to release the air in the airbag to an outside, a solenoid air valve has been utilized, the following configuration for which has been disclosed conventionally.

Japanese Patent Application Laid Open Publication No. 9-135817 discloses a flow rate control valve for controlling a flow rate by pressing an outflow route with elastic force of a spring of a valve seat provided at a tip of a moving plunger.

Further, Japanese Patent Application Laid Open Publication No. 2000-97364 discloses such a configuration that in a discharge valve for blocking a flow route by using an elastic member driven by a moving iron core or a moving coil, a space between this moving iron core or moving coil and a guide can be filled with a viscous member to control a flow rate smoothly.

In these air valves, an air outflow opening (nipple) is closed by pressing it with driving force strong enough to prevent leakage of air during pressurization and the elastic member (packing) is moved during measurement and at the end of measurement, thereby conducting depressurization control and air pressure releasing.

Further, recently, to improve portability and containment of a blood pressure monitor, a massager, etc., miniaturization of their bodies has been demanded, accompanied by a demand for further miniaturization of an air valve which is incorporated in them.

However, a structure of an air valve due to a conventional technology has a limit in miniaturization since coil or moving iron core is used. Further, such a structure has large power dissipation and generates a magnetic field and so that possibly affect its peripheral components.

If the moving iron core is thinned for the purpose of miniaturization, on the other hand, magneto-resistance increases to decrease driving force necessary for pressing of the packing and also it becomes difficult to arrange the packing covering the nipple portion sufficiently for closing the air outflow and inflow routes.

### SUMMARY OF THE INVENTION

In view of the above conventional technologies, the present invention has been developed, and it is an object of the present invention to provide a small-sized and simple air valve. It is another object of the present invention to provide a compact electronic blood pressure monitor equipped with the air valve. It is a further object of the present invention to provide a compact air massager equipped with the air valve.

To achieve that object, the present invention provides an air valve comprising:
an inflow opening through which air flows in;
an outflow opening through which the entered air flows out; and
an actuator having an elastomer or polymer that can expand and contract when supplied with a voltage and an electrode for applying a voltage on the elastomer or polymer,
wherein an outflow route from the inflow opening to the outflow opening is opened and closed by driving the actuator.

To this end, as an elastomer or a polymer that can be contracted and expanded when supplied with a voltage, a substance referred to as a dielectric elastomer or an electro-striction polymer (e.g., high-striction plastic having electric field response such as silicone resin, acrylic resin, or polyurethane) can be used. In particular, an electroactive polymer artificial muscle (EPAM) may be employed most suitably.

In contrast to a configuration of a solenoid air valve, this configuration need not use a coil or a moving iron core, thereby enabling simply providing a small-sized and lightweight structure of the apparatus.

Further, it is preferred that the actuator is provided with an elastic member, so that by causing the elastic member to come in contact with the inflow opening, the outflow route is blocked.

Further, it is preferred that the actuator is provided with an elastic member, so that by causing the elastic member to come in contact with the outflow opening, the outflow route is blocked.

Further, it is preferred that the actuator is provided with an elastic member at its end in its moving direction, so that by pressing the elastic member to a wall surface that forms the outflow route, the outflow route is blocked.

According to these configurations, it is possible to press an elastic member provided to an actuator to or bring it in contact with an inflow opening, an outflow opening, or a wall of an outflow route, thereby more precisely closing the outflow route.

Further, it is preferred that the air valve comprises a guide section for protecting an expansion/contraction section of the actuator and guiding its end on its moving side.

By this configuration, a guide section implements a protection function of the actuator and its guide function when it is expanded or contracted, thereby enabling stable opening and closing of the outflow route.

Further, it is preferred that the air valve comprises a restoration mechanism for generating restoring force on the actuator when it is contracted or expanded.

By this configuration, the actuator is equipped with a restoration mechanism for generating restoring force on the actuator when it is contracted or expanded, thereby enabling high-responsive opening and closing of the valve.

Further, it is preferred that an inflow direction in which gas flows in through the inflow opening and an outflow direction in which gas flows out through the outflow opening are different from each other.

Further, it is preferred that the inflow direction in which gas flows in through the inflow opening or the outflow direction in which gas flows out through the outflow opening is different from a direction in which the actuator expands and contracts.

Further, it is preferred that a direction in which the elastic member moves in order to block the outflow route is different from the direction in which the actuator expands and contracts.

By these configurations, the entire air valve can be thinned.

Further, it is preferred that a portion with which the actuator or the elastic member comes in contact in order to close the outflow route is provided with a projection.

By this configuration, it is possible to improve sealing with which the outflow route can be blocked.

Further, it is preferred that the actuator has a first elastomer or polymer that expands in such a direction as to block the outflow route when supplied with a voltage and a second elastomer or polymer that contracts in such a direction as to open the outflow route when supplied with a voltage; and
the outflow route is opened and closed by controlling the voltage applied on the first elastomer or polymer and the second elastomer or polymer.

By this configuration, a spring and a flexible member for generating restoring force are rendered unnecessary, thereby enabling reducing the number of components to be assembled and improving ease-to-assemble.

Further, it is preferred that a flow rate of a fluid that flows out through the outflow opening is controlled by controlling a voltage applied on the actuator or a frequency of the voltage.

By this configuration, by controlling a voltage (amplitude) or a frequency to be applied to the actuator, it is possible to control a degree of the actuator blocking the outflow route and pressing force with which the actuator or the elastic member comes in contact with the inflow or outflow opening, without changing a shape or a size of the member.

According to the present invention, there is provided an electronic blood pressure monitor comprising:
a fluid bag that is wound around a living body and filled with a fluid;
an air pump for sending the fluid to the fluid bag and pressuring it;
the air valve for controlling discharge of the fluid from the fluid bag;
a pressure sensor for detecting an inner pressure of the fluid bag; and
computation portion for performing processing to measure a blood pressure based on the detected inner pressure.

By this configuration, a coil or a moving iron core such as of a solenoid valve can be rendered unnecessary, thereby providing an electronic blood pressure monitor having a small-sized and/or simple configuration. Further, driving sound from, for example, an electromagnetic valve can be eliminated, to reduce noise when the air valve is being driven. In addition, it is possible to operate the apparatus with a smaller current than that required to drive the solenoid valve.

According to the present invention, there is provided an air massager comprising:
a seat;
a backrest section;
a plurality of airbags that is provided to the seat and/or backrest section and inflates and deflates when air comes in and goes out respectively; and
air control portion for controlling entrance of air to and its exit from each of the airbag,
wherein the air control portion has the air valve that is provided to each of the airbags.

Alternatively, according to the present invention, there is provided a rubbing air massager comprising:
a plurality of treatment portion for individually rubbing massaging at least two of the four limbs;
a plurality of airbags that is provided to the treatment portion and inflates and deflates when air comes in and goes out respectively; and
air control portion for controlling entrance of air to and its exit from the airbag,
wherein the air control portion has the air valve that is provided to each of the airbags.

By these configurations, a coil or a moving iron core such as of a solenoid valve can be rendered unnecessary, thereby providing an air massager having a simple configuration. Further, driving sound from, for example, an electromagnetic valve can be eliminated, to reduce noise when the air valve is being driven. In addition, it is possible to operate the apparatus with a smaller current than that required to drive the solenoid valve.

By the present invention, it is possible to realize a small-sized and simple air valve. Further, it is possible to realize a compact electronic blood pressure monitor equipped with the air valve. Still further, it is possible to realize a compact air massager equipped with the air valve.

### BRIEF DESCRIPTION OF THE DRAWINGS

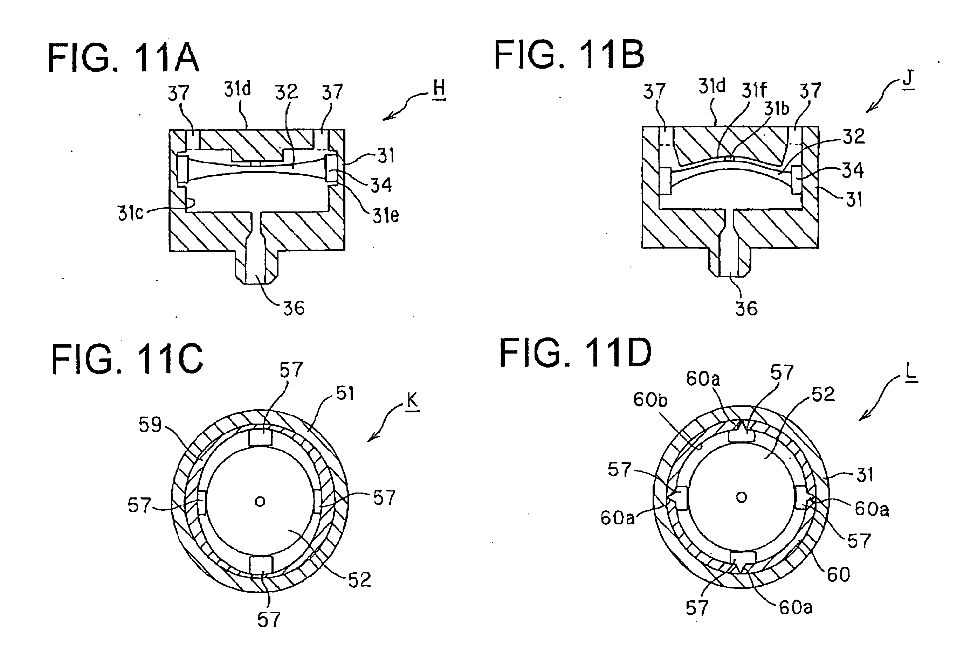

FIG. 1A is an external perspective view of an air valve related to a first embodiment, FIG. 1B is a cross-sectional view of the air valve related to the first embodiment in a condition where its outflow route is opened, and FIG. 1C is a cross-sectional view of the air valve related to the first embodiment in a condition where its outflow route is closed;
FIG. 2 is an explanatory schematic diagram of a structure of an EPAM;
FIG. 3 is a schematic diagram of how an actuator is deformed when it is supplied with a voltage;
FIG. 4 is a circuit diagram of a control circuit for controlling a voltage applied on the air valve;
FIG. 5A is a cross-sectional view of an air valve related to a second embodiment in a condition where its outflow route is opened and FIG. 5B is a cross-sectional view of the air valve related to the second embodiment in a condition where its outflow route is closed;
FIG. 6A is a cross-sectional view of an air valve related to a third embodiment in a condition where its outflow route is opened and FIG. 6B is a cross-sectional view of the air valve related to the third embodiment in a condition where its outflow route is closed;
FIGS. 7A and 7B are vertical and horizontal cross-sectional views of an air valve related to a fourth embodiment in a condition where its outflow route is opened and FIGS. 7C and 7D are vertical and horizontal cross-sectional views of the air valve related to the fourth embodiment in a condition where its outflow route is closed;
FIG. 8A is a cross-sectional view of an air valve related to a fifth embodiment in a condition where its outflow route is opened and FIG. 8B is a cross-sectional view of the air valve related to the fifth embodiment in a condition where its outflow route is closed;
FIGS. 9A and 9B are vertical and horizontal cross-sectional views of an air valve related to a sixth embodiment in a condition where its outflow route is opened and FIGS. 9C and 9D are vertical and horizontal cross-sectional views of the air valve related to the sixth embodiment in a condition where its outflow route is closed;
FIG. 10A is a cross-sectional view of an air valve related to a seventh embodiment in a condition where its outflow route is opened and FIG. 10B is a cross-sectional view of the air valve related to the seventh embodiment in a condition where its outflow route is closed;
FIG. 11A is a vertical cross-sectional view of an air valve related to an eighth embodiment in a condition where its outflow route is blocked, FIG. 11B is a vertical cross-sectional view of an air valve related to a ninth
embodiment in a condition where its outflow route is blocked, FIG. 11C is a horizontal cross-sectional view of an air valve related to a tenth embodiment in a condition where its outflow route is opened, and FIG. 11D is a horizontal cross-sectional view of an air valve related to an eleventh embodiment in a condition where its outflow route is opened;

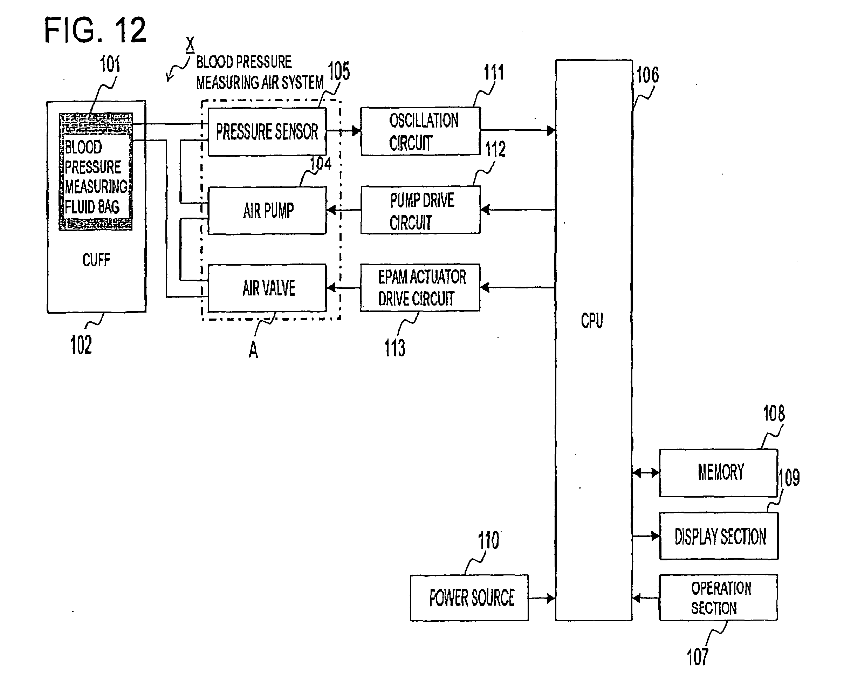

FIG. 12 is a block diagram of a hardware configuration of an electronic blood pressure monitor related to a twelfth embodiment;

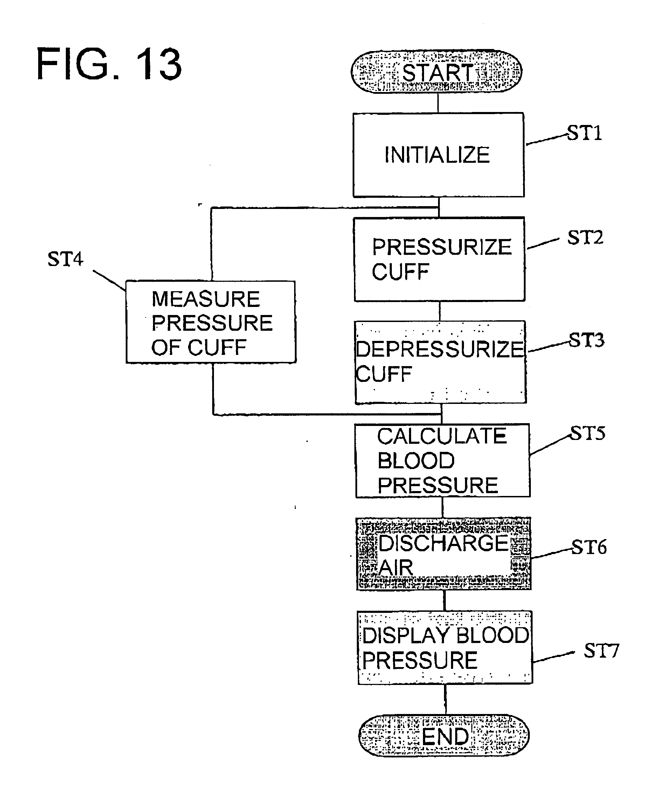

FIG. 13 is a flowchart of a basic operation of the blood pressure monitor related to the twelfth embodiment;

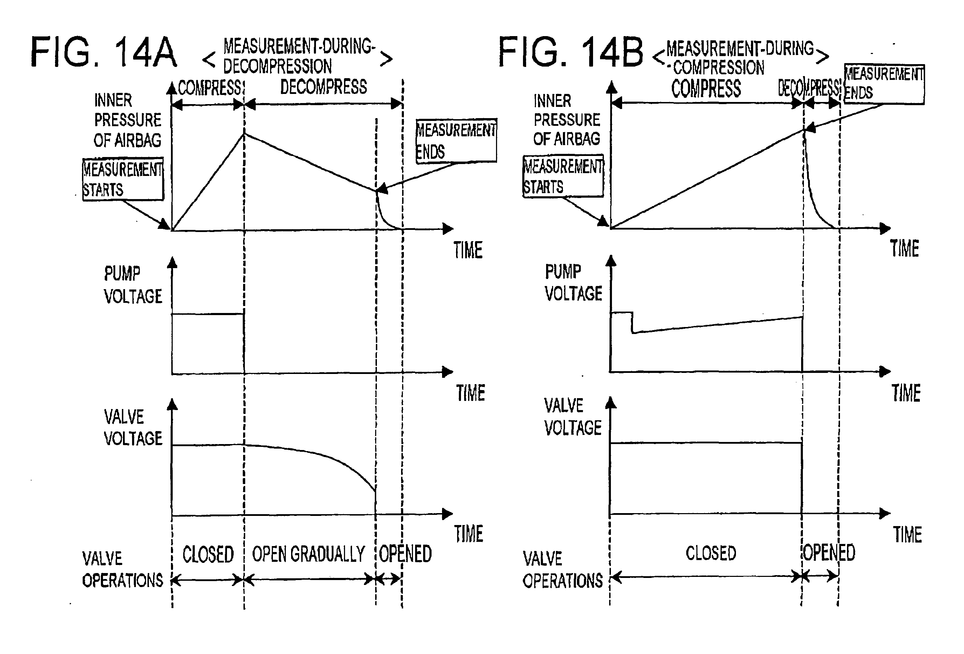

FIG. 14 is an explanatory schematic timing chart of changes in inner pressure of an airbag, pump voltage, and valve voltage at the time of measurement by the blood pressure monitor;

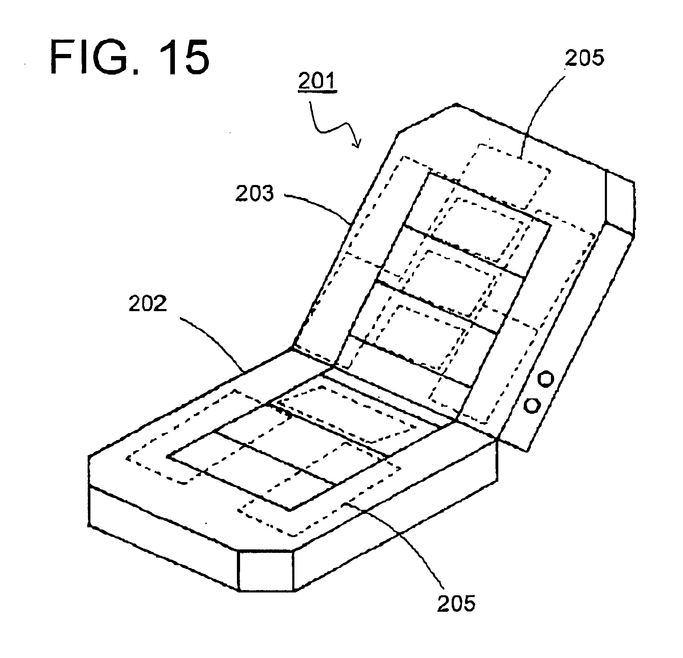

FIG. 15 is an external perspective view of a basic configuration of an air massager related to a thirteenth embodiment;

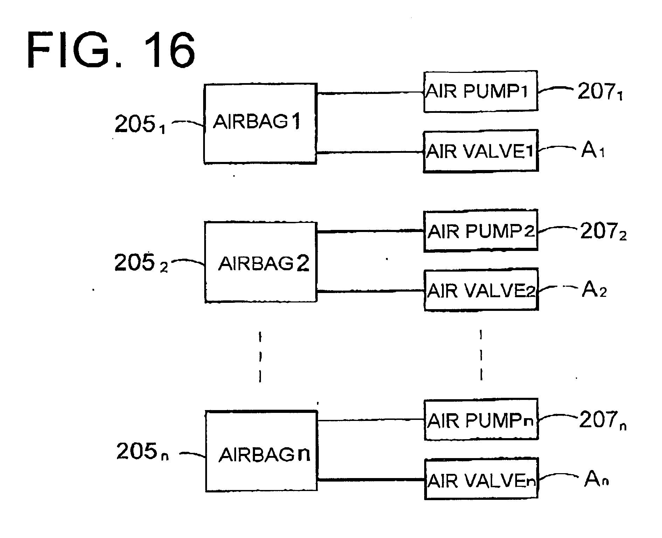

FIG. 16 is a block diagram of a configuration of the air massager related to the thirteenth embodiment;

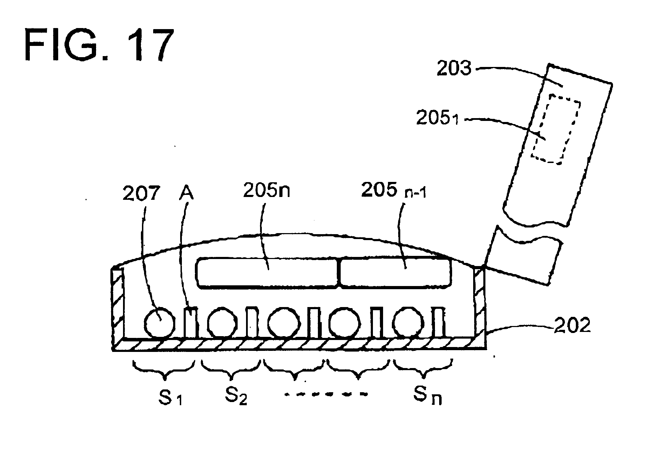

FIG. 17 is a cross-sectional view of primary components in the configuration of the air massager related to the thirteenth embodiment;

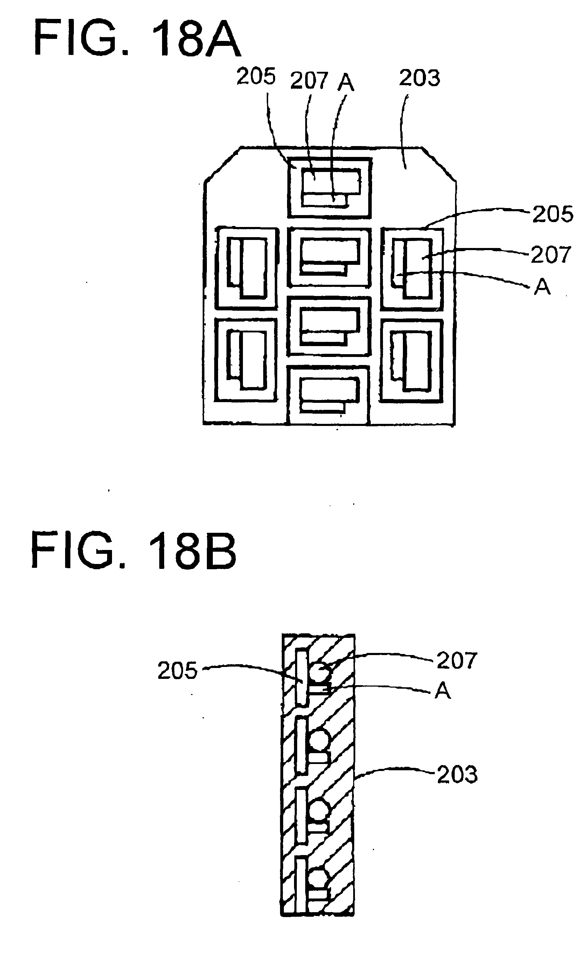

FIGS. 18 shows examples of arranging an air pump and a discharge valve in the configuration of the air massager related to the thirteenth embodiment, FIG. 18A of which is a rear view of a backrest section and FIG. 18B of which is a central vertical cross-sectional view of the backrest section as viewed from the right;

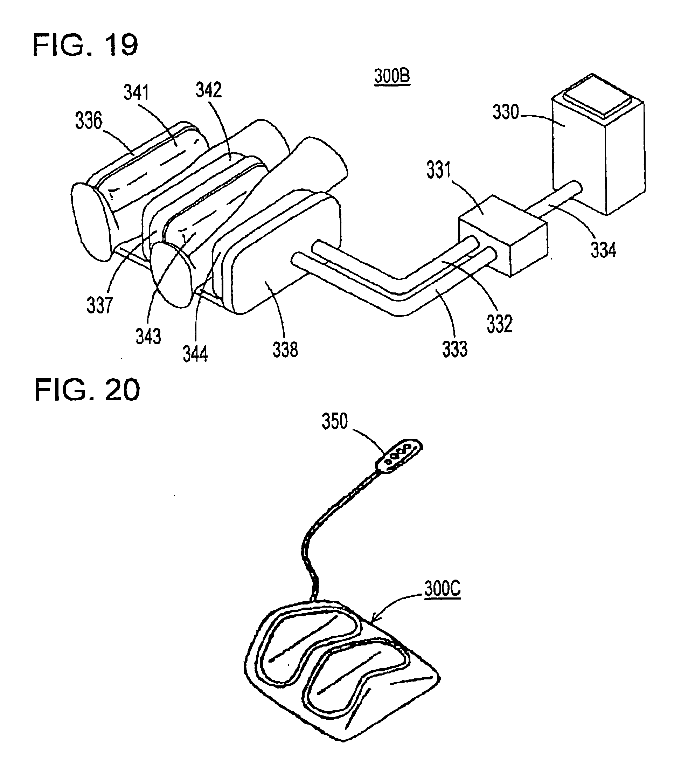

FIG. 19 is an external perspective view of a rubbing air massager related to a fourteenth embodiment;
FIG. 20 is a perspective view of one embodiment of a rubbing massager that uses an airbag related to the fourteenth embodiment;

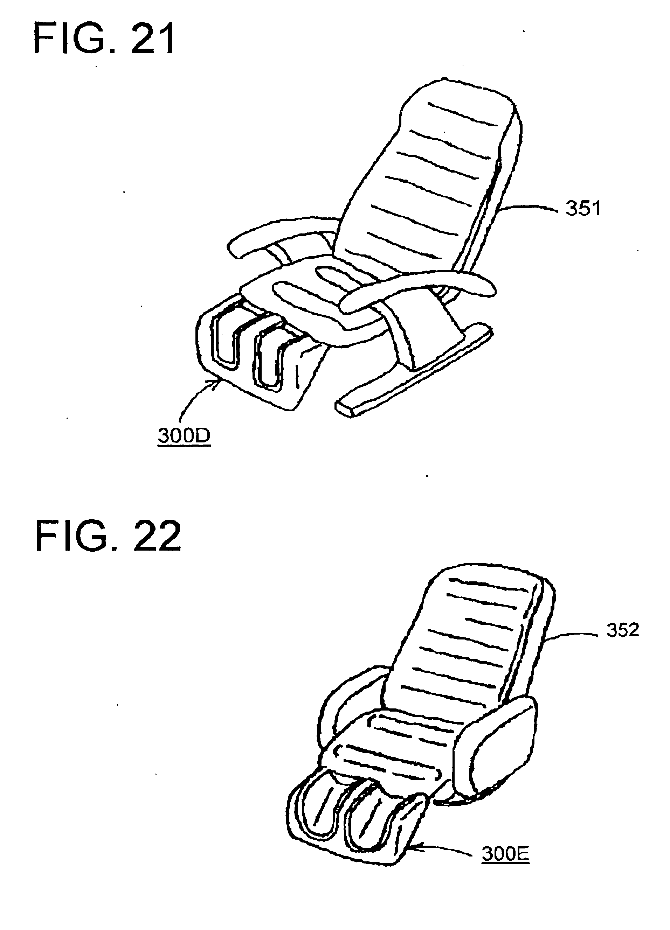

FIG. 21 is a perspective view of another embodiment of the rubbing massager that uses the airbag related to the fourteenth embodiment;
FIG. 22 is a perspective view of a further embodiment of the rubbing massager that uses the airbag related to the fourteenth embodiment;

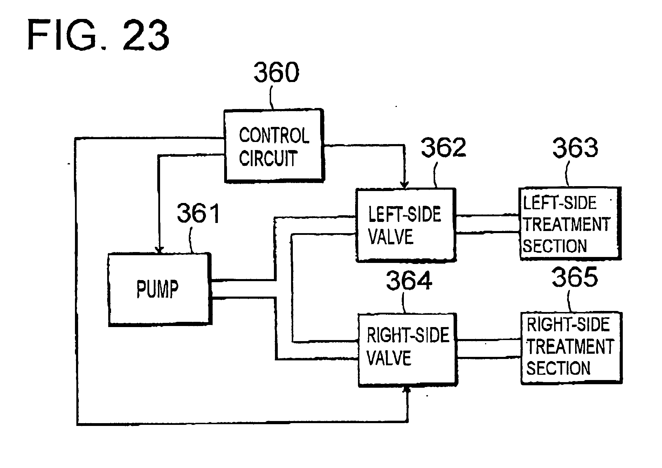

FIG. 23 is a block diagram of primary components of the rubbing massager that uses the airbag related to the fourteenth embodiment; and

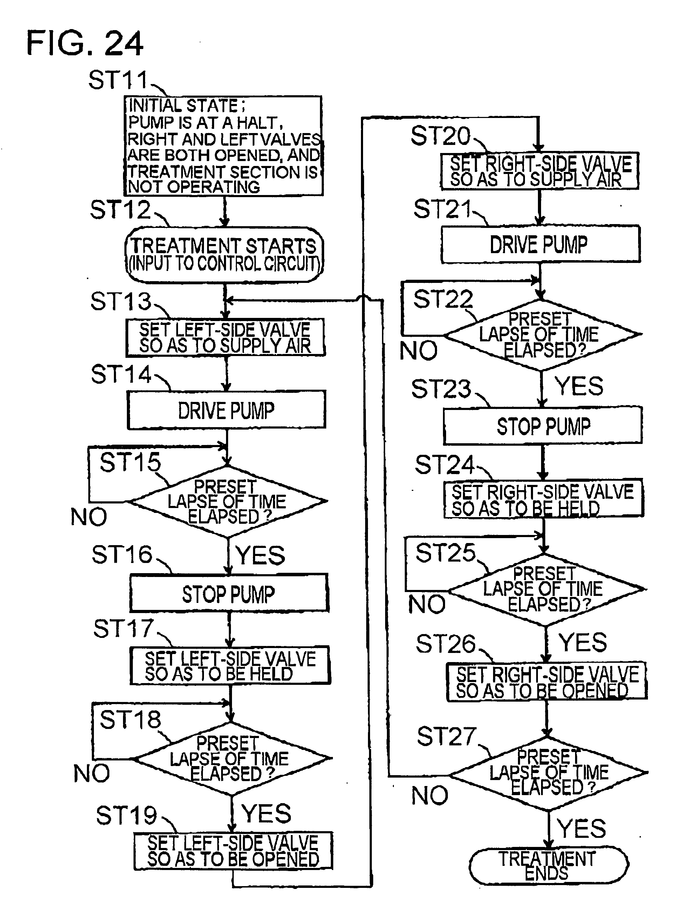

FIG. 24 is an explanatory flowchart of operations of the rubbing massager that uses the airbag related to the fourteenth embodiment.

### DETAILED DESCRIPTION OF THE PREFERRED EMBODIMENTS

The following will illustratively describe in detail the best mode for carrying out the present invention with reference to drawings and embodiments. However, a scope of the present invention is not intended to be limited to sizes, materials, shapes, functions, and relative arrangements of components described in these embodiments unless otherwise specified. Further, in the following description, the materials, the shapes, the functions, etc. of a member once explained are the same unless otherwise specified.

### [First Embodiment]

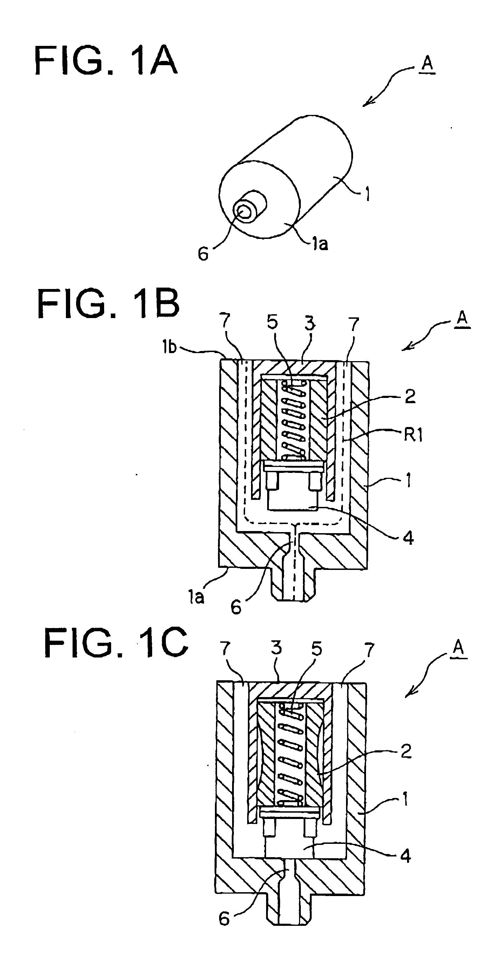

A configuration and a principle of an air valve related to the first embodiment are described with reference to FIG. 1. FIG. 1A is an external view of the air valve related to the first embodiment, FIG. 1B is a cross-sectional view of the air valve related to the first embodiment in a condition where its outflow route is opened, and FIG. 1C is a cross-sectional view of the air valve related to the first embodiment in a condition where its outflow route is closed.

The air valve A has a column-shaped housing member 1, a contractible/expandable actuator 2 provided in the housing member, a guide member 3 for guiding the actuator 2 so that it can be contracted and expanded in one direction, an elastic member 4 such as a packing provided to an end portion of the actuator 2 on its moving side, and a spring 5 provided as a flexible member in the cylinder-shaped actuator 2.

The guide member 3 protects the contractive/expansive section and guides its end portion on its moving side. It is thus possible to prevent the actuator 2 from being shifted from a desired position when it is being driven, thereby precisely opening and closing the air valve.

Further, the spring 5 made of elastic member (flexible member), which is one example of a restoration mechanism for generating restoring force against contraction and expansion of the actuator 2, is attached to the housing member 1 as supported thereby at a center of a face of the housing member 1 where the actuator 2 is attached. It is thus possible to open and close the air valve well responding to releasing of the actuator 2 of a voltage applied on it.

Further, one end face 1a of the housing member 1 has an inflow opening 6 formed therein for letting in gas discharged from any other member (e.g., airbag). Further, the other end face of the housing member 1 has one or a plurality of outflow openings 7 formed therein for discharging gas entered through the inflow opening 6. It is to be noted that as the any other member, an airbag used in a blood pressure monitor is described as an example.

Specifically, in a condition of FIG. 1B, an outflow route R1 from the inflow opening 6 to the outflow opening 7 is opened, so that an inner pressure of the airbag connected to the inflow opening 6 is equal to the atmospheric pressure.

It is to be noted that the actuator 2 has an elastomer or a polymer that can be contracted and expanded when supplied with a voltage and an electrode mounted for applying a voltage to the elastomer or the polymer.

Further, the elastomer or the polymer may employ an electroactive polymer artificial muscle (EPAM) to be described later most suitably, so that a case is described below, in each embodiment, where an EPAM is used in the actuator 2.

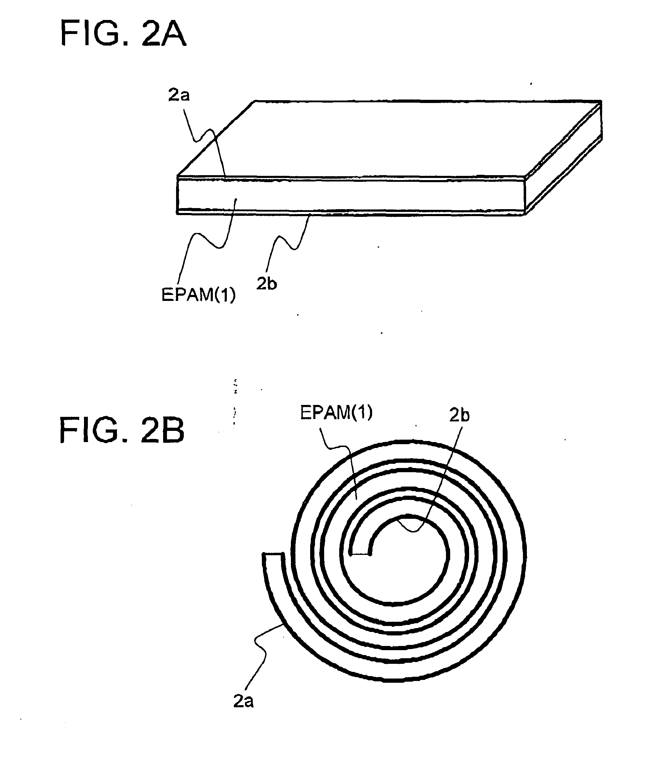

FIG. 2 is an explanatory schematic diagram of a structure of the EPAM employed most suitably in the present invention.

The actuator 2 related to the present embodiment is formed by winding up a film-shaped EPAM (1), such as shown in FIG. 2A, mounted on its both sides with contractive/expansive electrodes 2a and 2b respectively, one lap or a plurality of laps in a shape of a roll as shown in FIG. 2B. It is to be noted that when winding up the EPAM (1), to insulate the adjacent electrodes, an insulation material not shown is wound up together with the EPAM (1) in the shape of the roll.

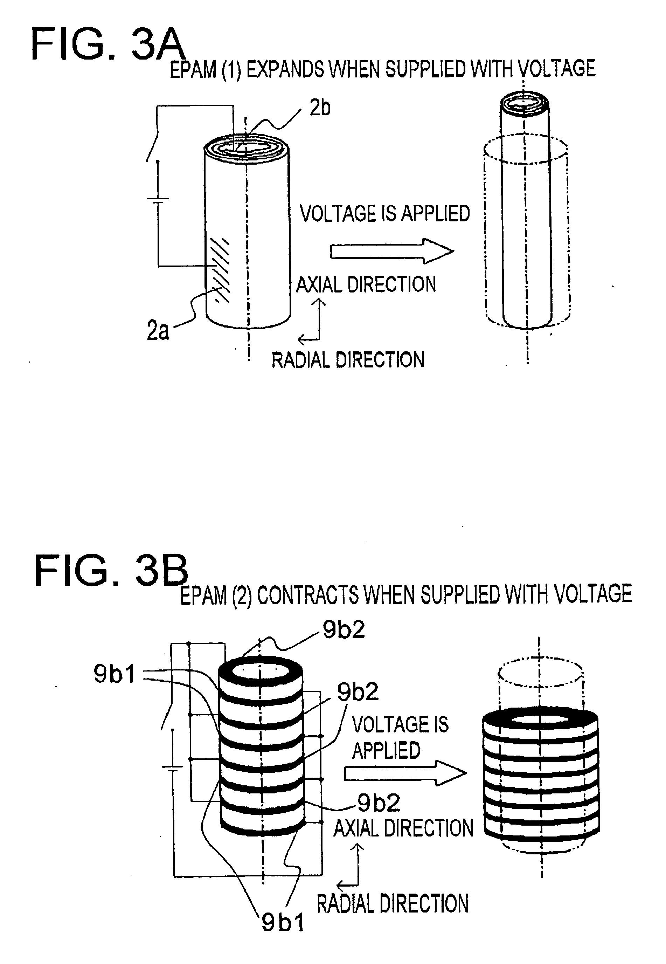

FIG. 3 is a schematic diagram of how the actuator employed most suitably in the present invention is deformed when it is supplied with a voltage.

As shown in FIG. 3A, when a voltage V is applied between its electrodes 2a and 2b, the actuator 2 related to the present embodiment is contracted radially and expanded axially. Therefore, in the aix valve A, as shown in FIG. 1C, the elasticmember 4 provided to the endportion on the moving side of the actuator 2 comes in contact with the inflow opening 6 to block it up, so that the outflow route R1 is blocked to prevent gas from entering through the airbag connected to the inflow opening 6, thereby enabling pressurization by use of a pump. Then, to release pressurization, the voltage applied to the actuator 2 is reduced to some value or zero to contract the actuator 2 axially owing to restoring force of the spring 5, so that the elastic member 4 is separated from the inflow opening 6, thereby opening the outflow route R1. That is, in the air valve A, the outflow route R1 from the inflow opening 6 to the outflow opening 7 can be opened and closed by driving the actuator 2.

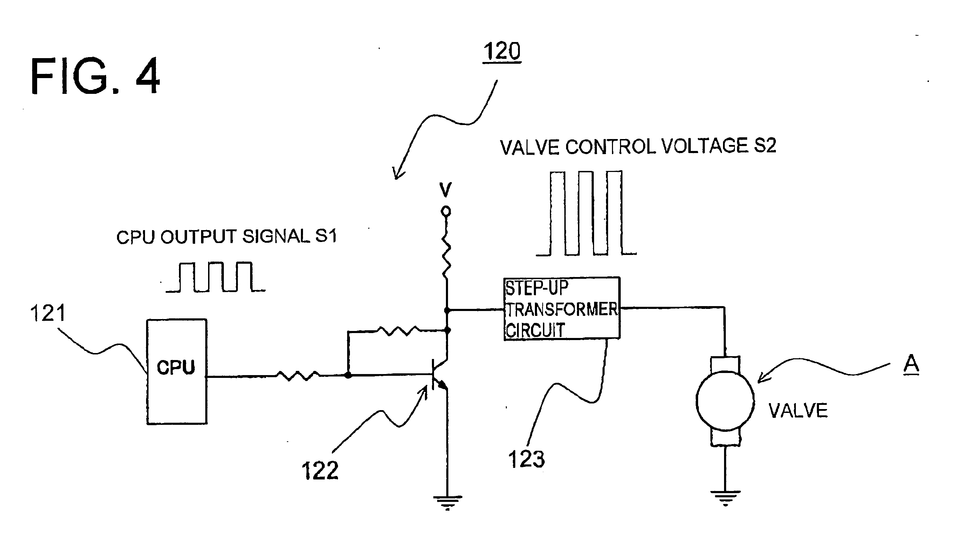

FIG. 4 is a circuit diagram of a control circuit 120 for controlling a voltage applied on the air valve A related to the present embodiment. When a CPU output signal S1 output from the CPU121 is input to a transistor 122, a voltage signal S2 in accordance with a wave shape (frequency and amplitude) of the CPU output signal S1 is generated. Then, the voltage signal S2 is input to a step-up transformer circuit 123 to be stepped up in voltage and input to the actuator 2 equipped to the air valve A. As a result, contraction and expansion of the actuator 2 can be controlled according to a magnitude of the input voltage, thereby opening and closing the air valve A. More specifically, by controlling a voltage or a frequency of the voltage applied to the actuator 2, it is possible to control an outflow rate from the outflow opening 7.

By thus using the above-described actuator 2, the air valve A related to the present embodiment can be reduced in size and weight with a simple configuration without an necessity of using a coil or a moving iron core in contrast to a solenoid air valve.

Further, the actuator 2, which uses an EPAM, can be changed in how much it contracts or expands (stroke) according to a magnitude of a voltage applied on it, so that by controlling a voltage (amplitude) or a frequency of the signal input to drive the actuator 2, force to press the elastic member 4 to the inflow opening 6 can be changed simply, thereby providing smooth flow rate control.

### [Second Embodiment]

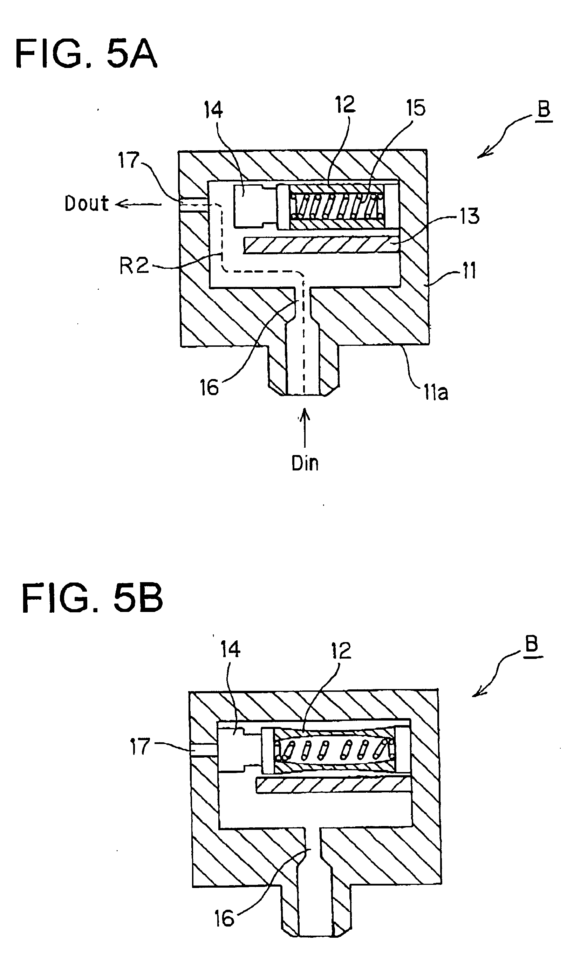

A configuration and an operation of an air valve related to the second embodiment are described with reference to FIG. 5. FIG. 5A is a cross-sectional view of the air valve related to the second embodiment in a condition where its outflow route is opened and FIG. 5B is a cross-sectional view of the air valve related to the second embodiment in a condition where its outflow route is closed.

An air valve B has a column-shaped housing member 11, a contractible/expandable actuator 12 provided in the housing member, a guide member 13 for guiding the actuator 12 so that it can be contracted and expanded in one direction, an elastic member 14 such as a packing provided to an end portion of the actuator 12 on its moving side, and a spring 15 provided in the cylinder-shaped actuator 12.

One end face 11a of the housing member 11 has an inflow opening 16 formed therein for letting in gas from an airbag. Further, a side face of the housing member 11 has one or a plurality of outflow openings 17 formed therein for discharging gas entered through the inflow opening 16. It is to be noted that in the air valve B related to the present embodiment, an inflow direction Din and an outflow direction Dout are different from each other.

In a condition of FIG. 5A, in the air valve B, an outflow route R2 from the inflow opening 16 to the outflow opening 17 is opened, so that an inner pressure of the airbag connected to the inflow opening 16 is equal to the atmospheric pressure.

It is to be noted that the actuator 12 can be made of the same EPAM (1) as that of the first embodiment. Further, the same control circuit as that of the first embodiment can be used to control a voltage applied to the air valve B.

As shown in FIG. 3A, when a voltage V is applied between the electrodes 2a and 2b, the actuator 12 related to the present embodiment is contracted radially and expanded axially. Therefore, in the air valve B, as shown in FIG. 5B, the elastic member 14 provided to the end portion on the moving side of the actuator 12 comes in contact with the outflow opening 17 to block it up, so that the outflow route R2 is blocked to prevent gas from being discharged from an inside of the air valve B, thereby enabling pressurization by use of a pump. Then, to release pressurization, the voltage applied to the actuator 12 is reduced to some value or zero to contract the actuator1 12 axially owing to restoring force of the spring 15, so that the elastic member 14 is separated from the outflow opening 17, thereby opening the outflow route R2.

In the air valve B related to the present embodiment, in contrast to the first embodiment, the outflow opening is not directed parallel with the inflow opening. Specifically, an inflow direction Din in which gas enters through the inflow opening 16 is at an angle of 90 degrees with respect to an outflow direction Dout in which the gas is discharged through the outflow opening 17. Such a configuration enables thinning the entire air valve B as compared to the air valve A related to the first embodiment in which the inflow direction and the outflow direction are parallel with each other.

Further, in the air valve B related to the present embodiment, the inflow direction Din is different from a direction in which the actuator 12 is contracted and expanded. Such a configuration enables thinning the entire air valve B as compared to the air valve A related to the first embodiment in which the inflow direction and the outflow direction are parallel with the direction in which the actuator is contracted and expanded.

By thus using the above-described actuator 12, the air valve B related to the present embodiment can be reduced in size and weight with a simple configuration without an necessity of using a coil or a moving iron core in contrast to a solenoid air valve.

Further, the actuator 12, which uses an EPAM, can be changed in how much it contracts or expands (stroke) according to a magnitude of a voltage applied on it, so that by controlling a voltage (amplitude) or a frequency of the signal input to drive the actuator 12, force to press the elastic member 14 to the outflow opening 17 can be changed simply, thereby providing smooth flow rate control.

### [Third Embodiment]

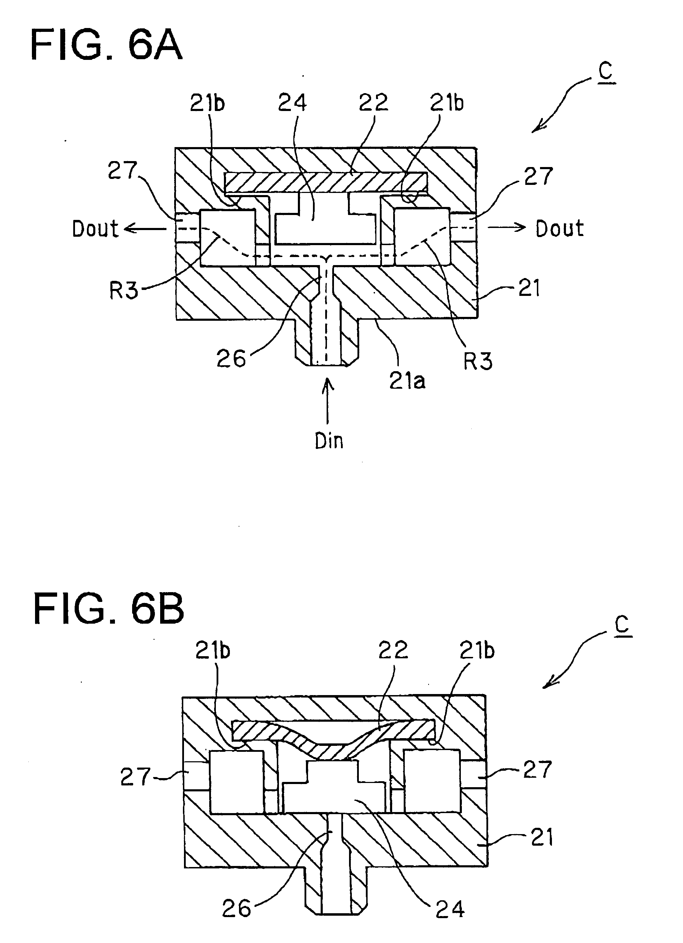

A configuration and an operation of an air valve related to the third embodiment are described with reference to FIG. 6. FIG. 6A is a cross-sectional view of the air valve related to the third embodiment in a condition where its outflow route is opened and FIG. 6B is a cross-sectional view of the air valve related to the third embodiment in a condition where its outflow route is closed.

An air valve C has a column-shaped housing member 21, a contractible/expandable plate-shaped actuator 22 provided in the housing member, a guide portion 21b for holding an edge portion of the actuator 22 so that the actuator 22 may not be deviated, and an elastic member 24 such as a packing provided at a center of one surface of the actuator 22.

One end face 21a of the housing member 21 has an inflow opening 26 formed therein for letting in gas from an airbag. Further, a side face of the housing member 21 has a plurality of outflow openings 27 formed therein for discharging gas entered through the inflow opening 26. It is to be noted that in the air valve C related to the present embodiment, an inflow direction Din and an outflow direction Dout are different from each other.

In a condition of FIG. 6A, in the air valve C, an outflow route R3 from the inflow opening 26 to the outflow opening 27 is opened, so that an inner pressure of the airbag connected to the inflow opening 26 is equal to the atmospheric pressure.

It is to be noted that the actuator 22 can be made of the same EPAM (1) as that of the first embodiment. Further, the same control circuit as that of the first embodiment can be used to control a voltage applied to the air valve C.

As shown in FIG. 2A, when a voltage V is applied between the electrodes 2a and 2b, the actuator 22 related to the present embodiment contracts in such a direction that a space between the electrodes 2a and 2b may be narrowed. The contracted EPAM (1) expands substantially perpendicular to the direction in which the space between the electrodes is narrowed. However, it cannot expand to an outside because the housing member 21 of the present invention comprises the guide portion 21b that holds the edge portion of the actuator 22. Therefore, the actuator 22 expands along its edge through its center, thereby permitting a middle of the actuator 22 to rise.

Accordingly, in the air valve C, as shown in FIG. 6B, the elastic member 24 provided at the middle of the actuator 22 comes in contact with the inflow opening 26 to block it up, so that the outflow route R3 is blocked to prevent gas from entering into the air valve C, thereby enabling pressurization by use of a pump. Then, to release pressurization, the voltage applied to the actuator 22 is reduced to some value or zero to restore the actuator 22 to its original shape owing to its own restoring force and the pressured gas, so that the elastic member 24 is separated from the inflow opening 26, thereby opening the outflow route R3. It is to be noted that the actuator 22 may not only be a circle but also a square in shape by devising an interior shape of the housing member 21.

In the air valve C related to the present embodiment, in contrast to the first embodiment, the outflow opening is not directed parallel with the inflow opening. Specifically, an inflow direction Din in which gas enters through the inflow opening 26 is at an angle of 90 degrees with respect to an outflow direction Dout in which the air is discharged through the outflow opening 27. Such a configuration enables thinning the entire air valve C as compared to the air valve A related to the first embodiment in which the inflow direction and the outflow direction are parallel with each other.

Further, in the configuration of the present embodiment, when blocking the outflow route R3, the actuator 22 does not contract and expand parallel with a direction in which the elastic member 24 moves to the inflow opening 26. Specifically, the direction in which the elastic member moves is at an angle of 90 degrees with respect to the direction in which the actuator 22 contract and expands. Such a configuration enables thinning the entire air valve C.

Further, in the air valve C related to the present embodiment, the inflow direction Din is different from a direction in which the actuator 22 is contracted and expanded. Such a configuration enables thinning the entire air valve C as compared to the air valve A related to the first embodiment in which the inflow direction and the outflow direction are parallel with the direction in which the actuator is contracted and expanded.

Further, by thus using the above-described actuator 22, the air valve C related to the present embodiment can be reduced in size and weight with a simple configuration without an necessity of using a coil or a moving iron core in contrast to a solenoid air valve.

Further, the actuator 22, which uses an EPAM, can be changed in how much it contracts or expands (stroke) according to a magnitude of a voltage applied on it, so that by controlling a voltage (amplitude) or a frequency of the signal input to drive the actuator 22, force to press the elastic member 24 to the inflow opening 26 can be changed simply, thereby providing smooth flow rate control.

### [Fourth Embodiment]

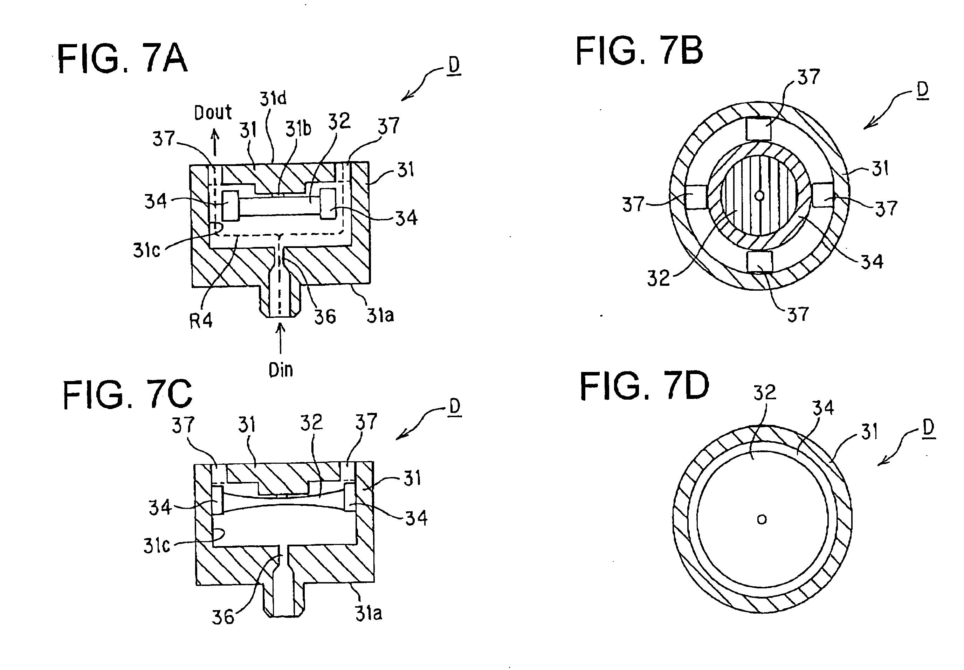

A configuration and an operation of an air valve related to the fourth embodiment are described with reference to FIG. 7. FIGS. 7A and 7B are vertical and horizontal cross-sectional views of an air valve related to the fourth embodiment in a condition where its outflow route is opened and FIGS. 7C and 7D are vertical and horizontal cross-sectional views of the air valve related to the fourth embodiment in a condition where its outflow route is closed.

An air valve D has a column-shaped housing member 31, a contractible/expandable disk-shaped actuator 32 provided in the housing member, a holding portion 31b for holding a middle portion of the actuator 32 so that the actuator 32 may not be deviated, and a ring-shaped elastic member 34 such as a packing provided at periphery of the actuator 32.

One end face 31a of the housing member 31 has an inflow opening 36 formed therein for letting in gas from an airbag. Further, the other end face 31d of the housing member 31 has a plurality of outflow openings 37 formed therein for discharging gas entered through the inflow opening 36.

In a condition of FIG. 7A and 7B, in the air valve D, an outflow route R4 from the inflow opening 36 to the outflow opening 37 is opened, so that an inner pressure of the airbag connected to the inflow opening 36 is equal to the atmospheric pressure.

It is to be noted that the actuator 32 can be made of the same EPAM (1) as that of the first embodiment. Further, the same control circuit as that of the first embodiment can be used to control a voltage applied to the air valve D.

As shown in FIG. 2A, when a voltage V is applied between the electrodes 2a and 2b, the actuator 32 related to the present embodiment contracts in such a direction that a space between the electrodes 2a and 2b may be narrowed. The contractedEPAM (1) expands perpendicular to the direction in which the space between the electrodes is narrowed. Therefore, a radius of the disk-shaped actuator 32 increases.

Accordingly, in the air valve D, as shown in FIGS. 7C and 7D, the ring-shaped elastic member 34 such as packing provided at the periphery of the actuator 32 comes in contact with or presses an internal circumference surface 31c of the housing member 31. That is, by providing the elastic member 34 at the end portion in the direction in which the actuator 32 moves and pressing the elastic member 34 to the internal circumference surface 31c as a wall surface that forms the outflow route R4, the outflow route R4 is blocked. In the present embodiment, the internal circumference surface 31c has a normal line different from the inflow and outflow directions. As a result, gas is not discharged from the air valve D, thereby enabling pressurization by use of a pump. Then, to release pressurization, the voltage applied to the actuator 32 is reduced to some value or zero to restore the actuator 32 to its original shape owing to its own restoring force, so that the elastic member 34 is separated from the internal circumference surface 31c, thereby opening the outflow route R4.

In the air valve D related to the present embodiment, the actuator 32 is configured to contract and expands in a direction different from an inflow direction Din and an outflow direction Dout. Specifically, the actuator 32 contracts and expands at an angle of 90 degrees with respect to the inflow direction Din and the outflow direction Dout. Such a configuration enables thinning the entire air valve D as compared to the air valve A related to the first embodiment in which the inflow direction and the outflow direction are parallel with the direction in which the actuator is contracted and expanded.

Further, by using the above-described actuator 32, the air valve D related to the present embodiment can be reduced in size and weight with a simple configuration without an necessity of using a coil or a moving iron core in contrast to a solenoid air valve.

Further, the actuator 32, which uses an EPAM, can be changed in how much it contracts or expands (stroke) according to a magnitude of a voltage applied on it, so that by controlling a voltage (amplitude) or a frequency of the signal input to drive the actuator 32, force to press the elastic member 34 to the internal circumference surface 31c can be changed simply, thereby providing smooth flow rate control. [Fifth Embodiment]

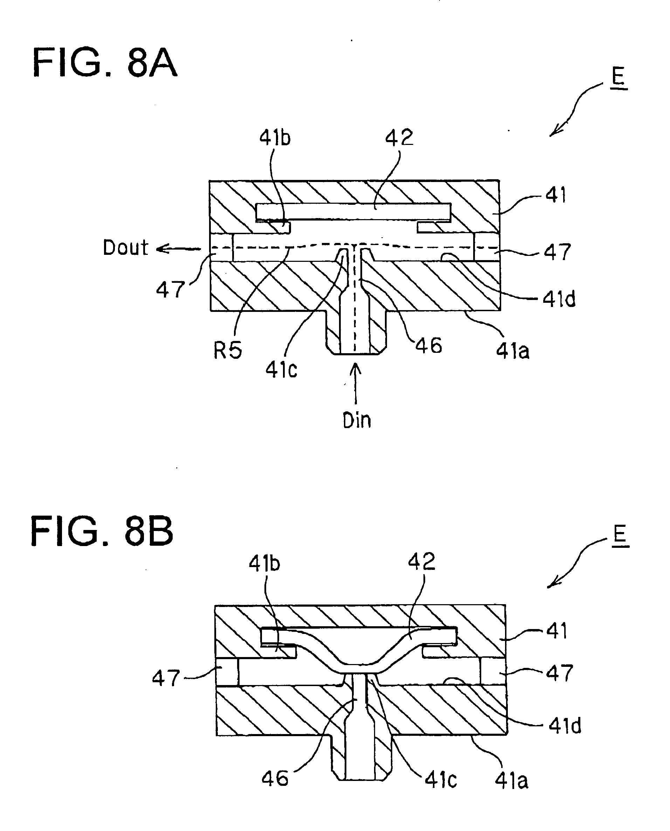

A configuration and an operation of an air valve related to the fifth embodiment are described with reference to FIG. 8. FIG. 8A is a cross-sectional view of an air valve related to the fifth embodiment in a condition where its outflow route is opened and FIG. 8B is a cross-sectional view of the air valve related to the fifth embodiment in a condition where its outflow route is closed.

An air valve E has a column-shaped housing member 41, a contractible/expandable plate-like actuator 42 provided in the housing member, and a guide portion 41b for holding an edge portion of the actuator 42 so that the actuator 42 may not be deviated.

One end face 41a of the housing member 41 has an inflow opening 46 formed therein for letting in gas from an airbag. Further, a side face of the housing member 41 has a plurality of outflow openings 47 formed therein for discharging gas entered through the inflow opening 46. It is to be noted that in the air valve E related to the present embodiment, an inflow direction Din and an outflow direction Dout are different from each other.

In a condition of FIG. 8A, in the air valve E, an outflow route R5 from the inflow opening 46 to the outflow opening 47 is opened, so that an inner pressure of the airbag connected to the inflow opening 46 is equal to the atmospheric pressure.

It is to be noted that the actuator 42 can be made of the same EPAM (1) as that of the first embodiment. Further, the same control circuit as that of the first embodiment can be used to control a voltage applied to the air valve E.

As shown in FIG. 2A, when a voltage V is applied between the electrodes 2a and 2b, the actuator 42 related to the present embodiment contracts in such a direction that a space between the electrodes 2a and 2b may be narrowed. The contracted EPAM (1) expands perpendicular to the direction in which the space between the electrodes is narrowed. However, it cannot expand to an outside because the housing member 41 of the present invention comprises the guide portion 41b that holds the edge of the actuator 42. Therefore, the actuator 42 expands along its edge portion through its center, thereby causing a middle portion of the actuator 42 to rise.

Accordingly, in the air valve E, as shown in FIG. 8B, a middle portion of the actuator 42 is pressed to a projection 41c that projects from an opposite inner face 41d of the housing member 41 toward the actuator 42. By providing such a projection 41c, sufficient sealing can be obtained even when the inflow opening 46 is blocked directly by the actuator 42; as a result, gas is not discharged from the air valve E, thereby enabling pressurization by use of a pump. Then, to release pressurization, the voltage applied to the actuator 42 is reduced to some value or zero to restore the actuator 42 to its original shape owing to its own restoring force and the pressured gas, so that the actuator 42 is separated from the inflow opening 46, thereby opening the outflow route R5. It is to be noted that the actuator 42 may not only be a circle but also a square in shape by devising an interior shape of the housing member 41.

In the air valve E related to the present embodiment, in contrast to the first embodiment, the outflow opening is not directed parallel with the inflow opening. Specifically, the inflow direction Din is at an angle of 90 degrees with respect to the outflow direction Dout. Such a configuration enables thinning the entire air valve E. Further, in contrast to the third embodiment, the present embodiment need not use an elastic member and therefore can realize a small-sized and lightweight air valve in a simpler configuration.

Moreover, the actuator 42 is configured not to contract or expand parallel with a direction in which the actuator 42 is deformed toward the inflow opening 46. Specifically, the middle portion of the actuator 42 moves at an angle of 90 degrees with respect to a direction in which the actuator 42 contracts and expands . Such a configuration enables thinning the entire air valve E.

Further, in the air valve E related to the present embodiment, the actuator 42 contracts and expands in a direction different from the inflow direction Din. Such a configuration enables thinning the entire air valve E as compared to the air valve A related to the first embodiment where the actuator contracts and expands parallel with the inflow and outflow directions.

Further, the air valve E related to the present embodiment uses the above-described actuator 42, to eliminate a necessity of using a coil or a moving iron core in contrast to a solenoid air valve, thereby enabling realizing a small-sized and light-weight apparatus in a simple configuration.

Further, the actuator 42, which uses an EPAM, can be changed in how much it contracts or expands (stroke) according to a magnitude of a voltage applied on it, so that by controlling a voltage (amplitude) or a frequency of the signal input to drive the actuator 42, force to press the actuator 42 to the inflow opening 46 can be changed simply, thereby providing smooth flow rate control.

### [Sixth Embodiment]

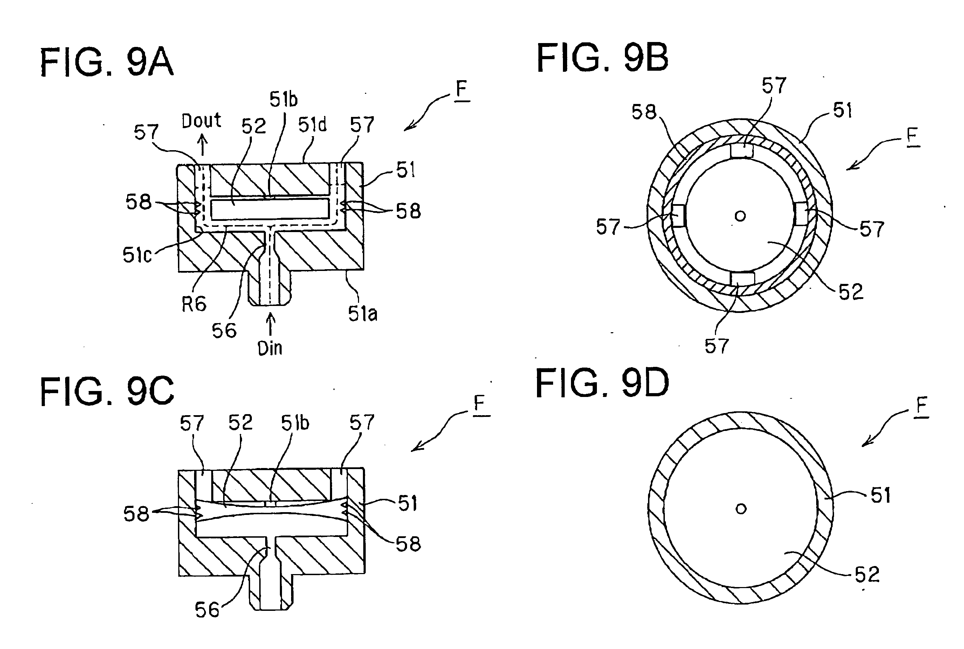

A configuration and an operation of an air valve related to the sixth embodiment are described with reference to FIG. 9. FIGS. 9A and 9B are vertical and horizontal cross-sectional views of an air valve related to the sixth embodiment in a condition where its outflow route is opened. FIGS. 9C and 9D are vertical and horizontal cross-sectional views of the air valve related to the sixth embodiment in a condition where its outflow route is closed.

An air valve F has a column-shaped housing member 51, a contractible/expandable disk-shaped actuator 52 provided in the housing member, and a holding portion 51b for holding a middle portion of the actuator 52 so that the actuator 52 may not be deviated.

One end face 51a of the housing member 51 has an inflow opening 56 formed therein for letting in gas from an airbag. Further, the other end face 51d of the housing member 51 has a plurality of outflow openings 57 formed therein for discharging gas entered through the inflow opening 56.

In conditions of FIGS. 9A and 9B, in the air valve F, an outflow route R6 from the inflow opening 56 to the outflow opening 57 is opened, so that an inner pressure of the airbag connected to the inflow opening 56 is equal to the atmospheric pressure.

It is to be noted that the actuator 52 can be made of the same EPAM (1) as that of the first embodiment. Further, the same control circuit as that of the first embodiment can be used to control a voltage applied to the air valve F.

As shown in FIG. 2A, when a voltage V is applied between the electrodes 2a and 2b, the actuator 52 related to the present embodiment contracts in such a direction that a space between the electrodes 2a and 2b may be narrowed. The contracted EPAM (1) expands perpendicular to the direction in which the space between the electrodes is narrowed. Therefore, a radius of the disk-shaped actuator 52 increases.

Accordingly, in the air valve F, as shown in FIGS. 9C and 9D, periphery of the actuator 52 comes in contact with or presses an internal circumference surface 51c of the housing member 51. The internal circumference surface 51c is provided with projections 58 annularly side by side which have a triangular cross section, so that by permitting the projections 58 to cut into an external circumference of the actuator 52, sufficient sealing can be obtained even when the outflow route R6 is blocked directly by the actuator 52. As a result, gas is not discharged from the air valve F, thereby enabling pressurization by use of a pump. Then, to release pressurization, the voltage applied to the actuator 52 is reduced to some value or zero to restore the actuator 52 to its original shape owing to its own restoring force, so that the actuator 52 is separated from the internal circumference surface 51c, thereby opening the outflow route R6.

In the air valve F related to the present embodiment, the actuator 52 is configured not to contract and expands parallel with an inflow direction Din and an outflow direction Dout. Specifically, the actuator 52 contracts and expands at an angle of 90 degrees with respect to the inflow direction Din and the outflow direction Dout. Such a configuration enables thinning the entire air valve F.

Further, by using the above-described actuator 52, the air valve F related to the present embodiment can be reduced in size and weight with a simple configuration without an necessity of using a coil or a moving iron core in contrast to a solenoid air valve.

Further, the actuator 52, which uses an EPAM, can be changed in how much it contracts or expands (stroke) according to a magnitude of a voltage applied on it, so that by controlling a voltage (amplitude) or a frequency of the signal input to drive the actuator 52, force to press the actuator 52 to the internal circumference surface 51c can be changed simply, thereby providing smooth flow rate control.

### [Seventh Embodiment]

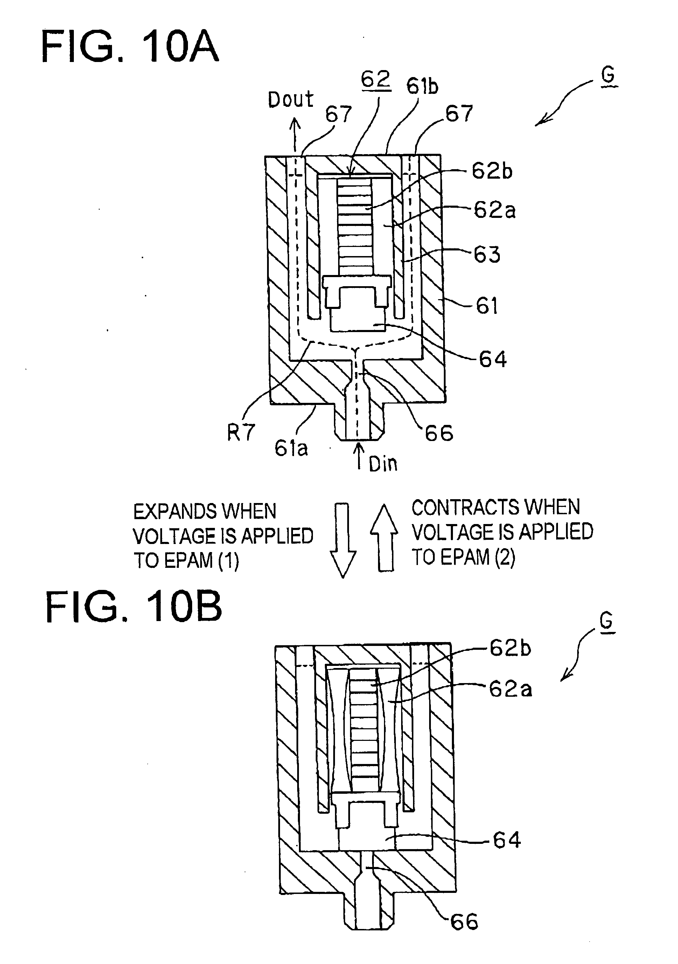

A configuration and a principle of an air valve related to the seventh embodiment are described with reference to FIG. 10. FIG. 10A is a cross-sectional view of the air valve related to the seventh embodiment in a condition where its outflow route is opened and FIG. 10B is a cross-sectional view of the air valve related to the seventh embodiment in a condition where its outflow route is closed.

An air valve G related to the seventh embodiment is different from the air valve A related to the first embodiment mainly in that it uses an actuator 62 that combines the EPAM (1) described in FIG. 3A with the EPAM (2) shown in FIG. 3B, thereby eliminating the spring. The following will describe differences in configuration, action, and effects, properly omitting the explanation of the same configurations as that of the first embodiment.

The EPAM (2) shown in FIG. 3B is mounted with electrodes 9b1 and 9b2 at its axial ends. In the present embodiment, in addition to them, the plurality of electrodes 9b1 and 9b2 is also arranged alternately along the EPAM (2). When a voltage V is applied between each pair of the electrodes 9b1 and 9b2, the EPAM (2) contracts axially and expands radially. Therefore, the EPAM (2) contracts vertically.

The air valve G has a column-shaped housing member 61, the contractible/expandable actuator 62 provided in the housing member, a guide member 63 for guiding the actuator 62 so that it can contract and expand only in one direction, and an elastic member 64 such as a packing provided at an end portion of the actuator 62 on its moving side.

As described above, the actuator 62 comprises a cylinder-shaped EPAM (1) 62a that expands in such a direction as to block an outflow route R7 when supplied with a voltage and a column-shaped or cylinder-shaped EPAM (2) 62b, which is arranged in its middle portion, that contracts in such a direction as to open the outflow route when supplied with a voltage.

In a condition of FIG. 10A, the outflow route R7 from an inflow opening 66 to an outflow opening 67 is opened, so that an inner pressure of an airbag connected to the inflow opening 66 is equal to the atmospheric pressure.

As shown in FIG. 3A, when a voltage V is applied across the EPAM (1) 62a, the actuator 62 related to the present embodiment contracts radially and expands axially. Accordingly, in the air valve G, as shown in FIG. 10B, the elastic member 64 provided at the end portion of the actuator 62 on its moving side blocks the inflow opening 66; as a result, air is not discharged from an airbag connected to the inflow opening 66, thereby enabling pressurization by use of a pump. Then, to release pressurization, the voltage applied across the EPAM (1) is released and, as shown in FIG. 3B, a voltage is applied across the EPAM (2) 62b so that the EPAM (2) 62b may contract axially and expand radially. Therefore, in the air valve G, as shown in FIG. 10A, it is possible to open the inflow opening 66 so that the outflow route R7 may be opened without using restoring force of an elastic member such as a spring.

In such a manner, by using the above-described actuator 62, the air valve G related to the present embodiment can be reduced in size and weight with a simple configuration without an necessity of using a coil or a moving iron core in contrast to a solenoid air valve.

Further, the actuator 62, which uses an EPAM, can be changed in how much it contracts or expands (stroke) according to a magnitude of a voltage applied on it, so that by controlling a voltage (amplitude) or a frequency of the signal input to drive the actuator 62, force to press the elastic member 64 to the inflow opening 66 can be changed simply, thereby providing smooth flow rate control.

Further, since the actuator 62 is mounted with the two EPAMs (1) 62a and (2) 62b side by side which contract and expand in different directions when supplied with a voltage, by controlling the respective application of voltages, it is possible to open and close the outflow route R7 in the air valve G without using an elastic member such as a spring, thereby reducing the number of components.

### [Eighth Embodiment]

A configuration of an air valve related to the eighth embodiment is described with reference to FIG. 11. FIG. 11A is a vertical cross-sectional view of the air valve related to the eighth embodiment in a condition where its outflow route is blocked.

An air valve H is greatly different from the air valve D related to the fourth embodiment in that it has a groove-shaped annular recess 31e formed in the internal circumference surface 31c of the housing member 31 which recess has a larger radius than an inner radius of the internal circumference surface 31c.

Since such an annular recess 31e is formed, even if force toward the outflow opening 37 is applied to the actuator 32 owing to compressed air entered through the inflow opening 36 during pressurization by use of a pump, it is possible to prevent the actuator 32 from being bent backward because the elastic member 34 is engaged with the annular recess 31e. As a result, good sealing can be obtained. Although the present embodiment has been described with reference to a configuration where the elastic member is used, the present embodiment can of course be applied to a configuration where the outflow route is blocked only with the actuator.

### [Ninth Embodiment]

A configuration of an air valve related to the ninth embodiment is described with reference to FIG. 11. FIG. 11B is a vertical cross-sectional view of an air valve related to the ninth embodiment in a condition where its outflow route is blocked.

An air valve J is greatly different from the air valve D related to the fourth embodiment in that a back-side inner face 31f of the other end face 31d of the housing member 31 provided with the holding portion 31b for holding an middle portion of the actuator 32 is a concave surface that is curved inwards in the middle from the outflow opening 37.

Since such a concave inner face 31f is formed, even if force toward the outflow opening 37 is applied to the actuator 32 owing to compressed air entered through the inflow opening 36 during pressurization by use of a pump, the actuator 32 is only deformed along the concave inner face 31f and can be prevented from being bent backward toward the outflow opening 37. As a result, good sealing can be obtained. Although the present embodiment has been described with reference to a configuration where the elastic member is used, the present embodiment can of course be applied to a configuration where the outflow route is blocked only with the actuator.

### [Tenth Embodiment]

A configuration of an air valve related to the tenth embodiment is described with reference to FIG. 11. FIG. 11C is a horizontal cross-sectional view of an air valve related to the tenth embodiment.

An air valve K is greatly different from the air valve F related to the sixth embodiment in that an annular projection 59 is not a perfect circle but an ellipsoid in shape.

Since the projection 59 is thus made ellipsoidal, when the actuator 52 is spread equally, first the actuator 52 comes in contact with the projection 59 provided on an internal circumference surface in a minor axis direction of the ellipsoid, to thereby close an outflow route partially, and then, the actuator 52 gradually comes in contact with the projection 59 provided on an internal circumference surface in a major axis direction of the ellipsoid. Accordingly, the outflow route is closed gradually, until the air valve K is closed finally. Such a configuration makes it possible to finely adjust an area of an opening formed between the actuator and the internal circumference surface with the same operations of the actuator as compared to the case of using the air valve D related to the fourth embodiment, so that by holding the actuator 52 as deformed in this condition, air can be discharged a little. Then, by further continuing deformation of the actuator 52, all of the outflow routes are closed, to enable pressurization by use of a pump also. Although the present embodiment has been described with reference to a configuration where only the actuator is used, the present embodiment can of course be applied to a configuration where the actuator is provided with an elastic member on its periphery.

### [Eleventh Embodiment]

A configuration of an air valve related to the eleventh embodiment is described with reference to FIG. 11. FIG. 11D is a horizontal cross-sectional view of the air valve related to the eleventh embodiment.

An air valve L is greatly different from the air valve F related to the sixth embodiment in that a projection 60 has a triangular notch 60a formed at part thereof.

Since a notch is formed at part of the projection 60, when the actuator 52 is spread equally, first the actuator 52 comes in contact with an internal circumference 60b of the projection 60 provided on an internal circumference surface of the housing member 31, to thereby close an outflow route mostly. In this case, the notch in the projection 60 is not blocked. Such a configuration makes it possible to finely adjust an area of an opening formed between the actuator and the internal circumference surface with the same operations of the actuator as compared to the case of using the air valve D related to the fourth embodiment, so that by holding the actuator 52 as deformed in this condition, air can be discharged a little. Then, by further continuing deformation of the actuator 52, all of the outflow routes are closed, to enable pressurization by use of a pump also. Although the present embodiment has been described with reference to a configuration where only the actuator is used, the present embodiment can of course be applied to a configuration where the actuator is provided with an elastic member on its periphery.

### [Twelfth Embodiment]

The following will describe an electronic blood pressure monitor according to the twelfth embodiment that can employ the above-described air valve suitably. FIG. 12 is a block diagram of a hardware configuration of the electronic blood pressure monitor. Although the present embodiment is described with reference to an example where the air valve A of the first embodiment is employed, the present embodiment can of course be applied to the air valves B to L of the respective second to eleventh embodiments.

### (Configuration of blood pressure monitor)

An electronic blood pressure monitor X comprises a fluid bag 101 which is wound around the upper arm (living body) when measuring a blood pressure, a compression/fixation cuff 102 for compressing and fixing this fluid bag 101 from circumference, an air pump 104 for sending a fluid such as air into the fluid bag 101 and pressuring it, an air valve A for controlling discharge of the fluid from the fluid bag 101, a pressure sensor 105 for detecting an inner pressure of the fluid bag 101, a CPU 106 as computation portion for performing processing to measure a blood pressure according to an internally stored program based on a detected inner pressure, an operation section 107 for making measurement settings and starting measurement, a memory 108 for storing set data, computation data, measurement results, etc., a display section 109 for displaying a set state, measurement results, etc., and a power source 110 for supplying electricity to these sections.

Further, the CPU 106 detects an inner pressure of the fluid bag 101 based on a signal output from the pressure sensor 105 and converted by an oscillation circuit 111. Then, if pressurization is necessary, the CPU 106 causes a pump drive circuit 112 to drive the air pump 104, thereby increasing an inner pressure of the fluid bag 101. If depressurization is necessary, on the other hand, it causes an EPAM actuator drive circuit 113 to open the air valve A, thereby decreasing the inner pressure of the fluid bag 101.

### (Basic operation of blood pressure monitor)

FIG. 13 is a flowchart of the basic operation of the blood pressure monitor to which the present invention can be applied suitably. Although it is described with reference to a case where the upper arm of the human body is measured, the present invention can be applied also to a living body which is not a human body, and measurement of other sites such as the wrist or the ankle can be possible. Although the flowchart of FIG. 13 shows the case of measurement-during-decompression by which a blood pressure is measured in a process of decompression after the site is compressed, the present invention can be applied to a blood pressure monitor for performing measurement-during-compression if the valve involves only ON/OFF operations.

First, when the cuff is wound around the upper arm (living body) and then power is turned ON to start the operation, initialization is performed to reset various settings of the electronic blood pressure monitor X to an initial state (step ST1).

The fluid 101 bag wound around the upper arm (living body) is pressurized to a predetermined pressure by the air pump 104 (step ST2). Simultaneously, a signal indicative of fluctuations in pressure of the fluid bag 101 detected by the pressure sensor 105 is transmitted via the oscillation circuit 111 to the CPU 106, which in turn starts measurement based on the signal (step ST4) .

After pressurization is completed, the inner pressure of the fluid bag 101 is gradually depressurized by opening the air valve A (step ST3) . Simultaneously, a signal indicative of fluctuations in pressure of the fluid bag 101 detected by the pressure sensor 105 is transmitted via the oscillation circuit 111 to the CPU 106, which in turn calculates systolic and diastolic blood pressures and a pulse rate (step ST5).

When the measurement is finished, the fluid bag 101 that has compressed the upper arm is evacuated of air through the air valve A, to decompress the upper arm (step ST6).

Next, the calculated blood pressure values etc. are displayed on the display section 109 (step ST7), to end a one-cycle measurement operation.

### (Changes in inner pressure of air bag, pump voltage, and valve voltage)

FIG. 14 is an explanatory schematic timing chart of changes in inner pressure of an airbag, pump voltage, and valve voltage at the time of measurement by the blood pressure monitor.

FIG. 14A shows an operation when measuring a blood pressure during decompression. In the case of measurement-during-decompression, almost simultaneously with start of measurement, a voltage is applied to the valve to close it. At the same time, the pump is driven to send air into the airbag so that its inner pressure may increase. When it is pressured sufficiently, the voltage applied on the pump is released to stop driving the pump. At the same time, the valve is gradually opened to depressurize the airbag at a constant speed. During the depressurization, a pulse wave is extracted from a change in inner pressure of the airbag, to calculate a blood pressure. When measurement is finished, the valve is opened completely to rapidly discharge air from the airbag, thereby releasing the human body.

FIG. 14B, on the other hand, shows an operation when measuring a blood pressure during compression. In the case of measurement-during-compression, almost simultaneously with start of measurement, a voltage is applied to the valve to close it. A voltage applied on the pump is controlled to adjust air sent into the airbag, thereby pressuring the airbag at a constant speed. A pulse wave is extracted from a change in inner pressure of the airbag being pressurized, to calculate a blood pressure. When measurement is finished, the valve is opened completely to rapidly discharge air from the airbag, thereby releasing the human body.

In such a manner, it is possible to employ an air valve of the present invention to a blood pressure monitor, thereby realizing a small-sized, lightweight, and simple configuration.

### [Thirteenth Embodiment]

The following will describe an air massager according to the thirteenth embodiment that can employ the above-described air valve suitably. FIG. 15 is an external perspective view of a basic configuration of the air massager related to the thirteenth embodiment. Although the present embodiment is described with reference to an example where the air valve A of the first embodiment is employed, the present embodiment can of course be applied to the air valves B to L of the respective second to eleventh embodiments.

### (Configuration of air massager)

An air massager 201, similar to an ordinary legless chair, apparently comprises a seat 202 and a backrest section 203 in such a configuration that a plurality of airbags 205 that inflates and deflates when it is supplied with and evacuated of air respectively is provided in the seat 202 and the backrest section 203, to each of the airbags 205 an air pump 207, not shown, being connected. The air massager 201 further comprises air control portion (not shown) for controlling of entrance of air to and its exit from the airbag 205.

In the present embodiment, the airbags 205 are all rectangular in shape, three of which are provided to the seat 202 and eight of which are provided to the backrest section 203. It is to be noted that the airbags 205 need not be rectangular in shape but may be circular, triangular, ellipsoidal, etc. appropriately and also may be increased or decreased in number according to a size and a shape thereof appropriately. In this air massager 201, by sending air to and releasing it from the airbags 205 by using the air control portion, the airbags 205 are inflated/deflated, thereby massaging the human body.

Besides the above fundamental configuration, this air massager 201 may possibly come in various modes, one example of which is described. FIG. 16 is a block diagram of primary components of the air massager. A configuration shown in FIG. 16 comprises the air pump 207 and the air valve A in which the air control portion is provided to each of the airbags. In FIG. 16, a total of n number of airbags 2051, 2052, ..., 205n are provided over the seat 202 and the backrest section 203 of the air massager 201 and, for each of the airbags, air pumps 2071, 2072, ... , 207n and air valves A1, A2, .., An are provided, thereby constituting the air control portion by these air pumps 207 and the air valves A.

In FIG. 16, to the airbags 2051, 2052, ..., 205n, the air pumps 2071, 2072, .., 207n and the air valves A1, A2, ..., An correspond respectively, so that to inflate the airbag 2051, for example, air can be sent to the airbag 2051 by operating the air pump 2071 in a condition where the air valve A1 is closed until the airbag encounters a predetermined pressure, whereupon the air pump 2071 is stopped. To deflate the airbag 2051, the airbag 2051 can be evacuated of air by opening the air valve A1.

### (Other configurations of chair massager)

FIG. 17 is a cross-sectional view of primary components of another configuration of the air massager 201, in which the air pumps 207 (2071, 2072, ..., 207n) and the air valves A (A1, A2, ..., An) that correspond to the n number of airbags 2051, 2052, ..., 205n are arranged in the seat 202. That is, unit S1 combining the air pump 2071 and the air valve A1 that correspond to the airbag 2051, ... , unit Sn similarly combining the air pump 207n and the air valve An that correspond to the airbag 205 are arranged sequentially. In this case, the air pump 207 and the air valve A which are main source of noise are distant from an upper part of the backrest section 203 to which the head (ear) of a person comes close, so that noise from the air pump 207 is suppressed for a user.

Further, by arranging the air pump 207 and the air valve A close to the corresponding airbag 205, a length of a passage (e.g., tube) that connects the airbag 205 and the air pump 207 to each other can be reduced to suppress deterioration in air pressure along the passage, so that air can be sent efficiently from the air pump 207 to the airbag 205, thereby improving response of inflation/deflation of the airbag 205.

FIGS. 18 show specific examples of arranging the air pumps 207 and the air valves A, FIG. 18A of which is a rear view of the backrest section 203 and FIG. 18B of which is a central vertical cross-sectional view of the backrest section 203 as viewed from the right. In this configuration, the air pumps 207 and the air valves A are arranged on the side opposite (rear side) to a human-body contacting face of the airbag 205 and the corresponding air pumps 207 and air valves A are arranged at an upper part and a lower part of the rear side of the airbags 205 respectively. This layout is the same for all of the airbags 205 provided to the backrest section 203. Of course, they may be arranged similarly in the seat 202. By arranging them in a depth direction of the air massager 201, it is possible to secure a large human-body contacting face of the backrest section 203, thereby increasing a degree of freedom in arrangement of the airbags 205 to the human-body contacting face.

By the present embodiment, the airbags provided to the air massager are inflated/deflated using an air valve in which a coil or a moving iron core need not be used in contract to a solenoid air valve of the disclosed conventional technology, thereby enabling providing a simple configuration of the air massager. Further, it can be reduced in size and weight as compared to the case of employing an air pump that uses a solenoid valve.

Moreover, it is free of noise from the moving iron core to reduce noise when the air valve is being driven, thereby suppressing uncomfortable feeling of the user. In addition, the apparatus can be operated with a smaller current than that required to drive a solenoid.

### [Fourteenth Embodiment]

The following will describe an rubbing air massager according to the fourteenth embodiment that can employ the above-described air valve suitably. An external perspective view of a rubbing air massager related to the fourteenth embodiment is shown in FIG. 19. Although the present embodiment is described with reference to an example where the air valve A of the first embodiment is employed, the present embodiment can of course be applied to the air valves B to L of the respective second to eleventh embodiments.

By this rubbing air massager 300B also, air bag pairs 341, 342, 343, and 344 are repeatedly inflated/deflated by turns as a plurality of treatment portion for individually rubbing or pressing massaging at least two of the four limbs, thereby providing rubbing or pressing actions and advantages equivalent to those described above.

A variety of embodiments of a rubbing air massager by use of an air bag are shown in FIGS. 20 to 22. A rubbing air massager 300C of FIG. 20, which is used as a discrete as it is, has a controller 350. An air massager 300D of FIG. 21, which is of a chair type, is attached to a chair 351, an air massager 300E of FIG. 22, which is of a legless chair type, is attached to a legless chair 352.

A block diagram of primary components of the rubbing air massagers 300B to 300E that use these airbags is shown in FIG. 23. A pump 361 is connected via a left-side air valve 362 and a right-side air valve 364 to a left-side treatment section (left-side one airbag pair) 363 and a right-side treatment section (right-side one airbag pair) 365 respectively in such a configuration that the pump 361 and the air valves 362 and 364 are controlled by a control circuit 360.

The following will describe operations of the rubbing air massager that uses an airbag having a configuration of FIG. 23 with reference to a flowchart of FIG. 24. First, at step ST11, in an initial state, the pump 361 is at a halt and the air valves 362 and 364 are both open, so that of course the right and left treatment sections 363 and 365 are not operating either. When a treatment start signal is input to the control circuit 360 as, for example, the user presses a starting switch, treatment starts (step ST12). In the present embodiment, first the left-side air valve 362 is set so that it may supply air (step ST13), to operate the pump 361 (step ST14). Accordingly, air is sent to the left-side treatment section 363, which continues until a preset lapse of time elapses (step ST15). When the preset lapse of time elapses, that is, the left-side treatment section 363 is inflated sufficiently, the pump 361 stops (step ST16) and the left-side air valve 362 is set to be held (step ST17), an inflated state of which continues until a preset lapse of time elapses (step ST18). When the preset lapse of time elapses, the left-side air valve 362 is opened (step ST19), to evacuate the left-side treatment section 363 of air, so that the left-side treatment section 363 is deflated.

While, on the other hand, the right-side air valve 364 is set to supply air (step ST20), to operate the pump 361 (step ST21), thereby inflating the right-side treatment section 365 with air. When a preset lapse of time elapses (step ST22) and the right-side treatment section 365 is inflated sufficiently, the pump 361 stops (step ST23), so that the right-side air valve 364 is set to be held (step ST24), an inflated state of which is held until a preset lapse of time elapses (step ST25). When the preset lapse of time elapses, the right-side air valve 364 is opened (step ST26), to evacuate the right-side treatment section 365 and deflate it. Subsequently, the process decides whether a preset lapse of time for the entire medical treatment has elapsed or not (step ST27), and if such is not the case, returns to step ST13 to repeat the same treatment operations and, otherwise, ends the treatment.

Although the present invention has been described with reference to the above embodiments, it is not limited to them; these embodiment can of course be modified and combined in configuration as much as possible.

## Claims

1. An air valve comprising:
an inflow opening through which air flows in;
an outflow opening through which the entered air flows out; and
an actuator having an elastomer or polymer that can expand and contract when supplied with a voltage and an electrode for applying a voltage on the elastomer or polymer,
wherein an outflow route from the inflow opening to the outflow opening is opened and closed by driving the actuator.

2. An air valve according to claim 1, wherein the actuator is provided with an elastic member, so that by causing the elastic member to come in contact with the inflow opening, the outflow route is blocked.

3. An air valve according to claim 1, wherein the actuator is provided with an elastic member, so that by causing the elastic member to come in contact with the outflow opening, the outflow route is blocked.

4. An air valve according to claim 1, wherein the actuator is provided with an elastic member at its end in its moving direction, so that by pressing the elastic member to a wall surface that forms the outflow route, the outflow route is blocked.

5. An air valve according to any one of claims 1 to 4, comprising a guide section for protecting an expansion/contraction section of the actuator and guiding its end on its moving side.

6. An air valve according to any one of claims 1 to 5, comprising a restoration mechanism for generating restoring force on the actuator when it is contracted or expanded.

7. An air valve according to any one of claims 1 to 6, wherein an inflow direction in which gas flows in through the inflow opening and an outflow direction in which air flows out through the outflow opening are different from each other.

8. An air valve according to any one of claims 1 to 7, wherein the inflow direction in which gas flows in through the inflow opening or the outflow direction in which gas flows out through the outflow opening is different from a direction in which the actuator expands and Contracts.

9. An air valve according to any one of claims 1 to 8, wherein a direction in which the elastic member moves in order to block the outflow route is different from the direction in which the actuator expands and contracts.

10. An air valve according to any one of claims 1 to 9, wherein a portion with which the actuator or the elastic member comes in contact in order to close the outflow route is provided with a projection.

11. An air valve according to any one of claims 1 to 10, wherein:
the actuator has a first elastomer or polymer that expands in such a direction as to block the outflow route when supplied with a voltage and a second elastomer or polymer that contracts in such a direction as to open the outflow route when supplied with a voltage; and
the outflow route is opened and closed by controlling the voltage applied on the first elastomer or polymer and the second elastomer or polymer.

12. An air valve according to any one of claims 1 to 11, wherein a flow rate of a fluid that flows out through the outflow opening is controlled by controlling a voltage applied on the actuator or a frequency of the voltage.

13. An electronic blood pressure monitor comprising:
a fluid bag that is wound around a living body and filled with a fluid;
an air pump for sending the fluid to the fluid bag and pressuring it;
the air valve according to any one of claims 1 to 12 for controlling discharge of the fluid from the fluid bag;
a pressure sensor for detecting an inner pressure of the fluid bag; and
computation portion for performing processing to measure a blood pressure based on the detected inner pressure.

14. An air massager comprising:
a seat;
a backrest section;
a plurality of airbags that is provided to the seat and/or backrest section and inflates and deflates when air comes in and goes out respectively; and
air control portion for controlling entrance of air to and its exit from each of the airbag,
wherein the air control portion has the air valve according to any one of claims 1 to 12 that is provided to each of the airbags.

15. An air massager comprising:
a plurality of treatment portion for individually rubbing massaging at least two of the four limbs;
a plurality of airbags that is provided to the treatment portion and inflates and deflates when air comes in and goes out respectively; and
air control portion for controlling entrance of air to and its exit from the airbag,
wherein the air control portion has the air valve according to any one of claims 1 to 12 that is provided to each of the airbags.
